# EUROPEAN PATENT SPECIFICATION

(11) **EP 2 918 287 B1**
(45) Date of publication and mention of the grant of the patent: **20.06.2018**
(21) Application number: 14000866.5
(22) Date of filing: 11.03.2014
(51) Int. Cl.: A61K 35/28, A61K 38/48, A61P 37/06, A61K 39/00

(54) **Pre-incubation of T cells**
Vorinkubation von T-Zellen
Lymphocytes T de pré-incubation

(43) Date of publication of application: 16.09.2015
(73) Proprietor: Otto-von-Guericke-Universität Magdeburg, 39106 Magdeburg (DE)
(72) Inventor: Isermann, Berend, 39128 Magdeburg (DE); Ranjan, Satish, 39120 Magdeburg (DE)
(74) Representative: Müller-Boré & Partner Patentanwälte PartG mbB

(56) References cited:
- WO-A1-2013/076268
- WO-A2-01/90342
- WO-A2-2005/007820
- CONTRERAS JUAN L ET AL: "Activated protein C preserves functional islet mass after intraportal transplantation - A novel link between endothelial cell activation, thrombosis, inflammation, and islet cell death", DIABETES, vol. 53, no. 11, November 2004 (2004-11), pages 2804-2814, XP003007163, ISSN: 0012-1797
- CONTRERAS J L ET AL: "ACTIVATED HUMAN PROTEIN C PROTECTS PANCREATIC ISLETS (HPI) FROM PROINFLAMMATORY CYTOKINE (PIC) DAMAGE: A NOVEL LINK BETWEEN COAGULATION, INFLAMMATION AND CELL DEATH", CELL TRANSPLANTATION, COGNIZANT COMMUNICATION CORP, US, vol. 12, no. 2, 1 January 2003 (2003-01-01), page 143, XP003007160, ISSN: 0963-6897

## Description

The present invention relates to an *ex vivo* method for the treatment of a bone marrow transplant, wherein the bone marrow transplant is treated *ex vivo* with activated Protein C (aPC) prior to implanting into the recipient and wherein the bone marrow transplant treatment is intended for use in preventing Graft versus Host Disease (GVHD) associated with transplantation.

Graft versus Host Disease is one of the major complications associated with transplantation. The presence of immune cells of the donor in the transplant result in an attack of the donor's immune cells against cells of the recipient after transplantation. The donor's immune cells recognize the recipient's cells as antigenetically different. Effector cells in the development of GVHD are T cells of the donor. While the acute form of GVHD is normally observed within the first 100 days after the transplantation, the chronic form of GVHD usually occurs after 100 days after the transplantation. Both forms appear to involve different immune cell subsets. At present there is no effective therapy available against GVHD.

The serine protease protein C is a naturally occurring anticoagulant. It plays a role in the regulation of vascular hemostasis. In particular, it inactivates Factor Va and Factor VIIIa in the coagulation cascade. The single polypeptide of protein C, which is primarily synthesized in the liver, is the subject of many post-translational modifications which result in a circulating zymogen with two chains. The activation of protein C to activated protein C (aPC) is carried out by thrombin in the presence of calcium ion and results in a removal of a dodecapeptide at the N-terminus of the heavy chain of protein C.

Thus, the problem underlying the present invention is to provide new means for an effective treatment of Graft versus Host Disease.

The solution to the above technical problem is achieved by the embodiments characterized in the claims.

In particular, the present invention relates to an *ex vivo* method for the treatment of a bone marrow transplant, wherein the bone marrow transplant is treated *ex vivo* with activated Protein C (aPC) prior to implanting into the recipient and wherein the bone marrow transplant treatment is intended for use in preventing Graft versus Host Disease (GVHD) associated with transplantation.

According to the present invention, the term "activated Protein C (aPC)" does not underlie a specific restriction and may include any aPC, aPC complex or polypeptide, including recombinant aPC, aPC complexes and polypeptides either isolated from a natural source or obtained via recombinant DNA technology, or a biologically active derivative thereof.

As used herein, the term "biologically active derivative" includes any derivative of aPC, aPC complex or polypeptide having substantially the same functional and/or biological properties of aPC such as binding properties, and/or the same structural basis, such as a peptidic backbone. The polypeptide sequences of the functionally active derivatives may contain deletions, additions and/or substitution of amino acids whose absence, presence and/or substitution, respectively, do not have any substantial negative impact on the activity of the polypeptide, e.g. amino acids which are located in a part of the polypeptide sequence that does not contribute to the biological activity of aPC. Minor deletions, additions and/or substitutions of amino acids of the respective polypeptide sequences which are not altering the biological activity of said polypeptide are also included in the present application as biologically active derivatives.

An aPC obtained from a natural source may be any aPC isolated from a blood product derived from a mammal. In a preferred embodiment of the present application, the mammal is selected from the group consisting of mouse, human, rat, cat, dog, and monkey.

The aPC may be derived from any mammal. In a preferred embodiment, the aPC is derived from a mammal is selected from the group consisting of mouse, human, rat, cat, dog, and monkey. According to the present invention, the aPC may be derived from the same species as the donor and/or the recipient or from a different species as the donor and/or the recipient.

The aPC according to the present invention may be produced by any method known in the art. This may include any method known in the art for the production of recombinant DNA by genetic engineering, e.g. via reverse transcription of RNA and/or amplification of DNA. This includes methods which comprise the recombinant production of aPC.

The recombinant aPC used according to the present invention may be produced by any method known in the art. This may include any method known in the art for (i) the production of recombinant DNA by genetic engineering, e.g. via reverse transcription of RNA and/or amplification of DNA, (ii) the introduction of recombinant DNA into prokaryotic or eukaryotic cells by transfection, e.g. via electroporation or microinjection, (iii) the cultivation of said transformed cells, e.g. in a continuous or batchwise manner, (iv) the expression of aPC, e.g. constitutive or upon induction, and (v) the isolation of aPC, e.g. from the culture medium or by harvesting the transformed cells, in order to (vi) obtain purified recombinant aPC, e.g. via anion exchange chromatography or affinity chromatography.

For example, the recombinant DNA coding for aPC, e.g. a plasmid, may also contain a DNA sequence encoding a selectable marker for selecting the cells which have been successfully transfected with the plasmid. In an example of the present invention, the plasmid may also confer resistance to a selectable marker, e.g. to the antibiotic drug G418, by delivering a resistance gene, e.g. the neo resistance gene conferring resistance to G418.

The production of aPC may include any method known in the art for the introduction of recombinant DNA into cells, which might be mammalian cells, bacterial cells or insect cells, by transfection, e.g. via electroporation or microinjection. For example, the recombinant expression of aPC can be achieved by introducing an expression plasmid containing aPC encoding DNA sequence under the control of one or more regulating sequences such as a strong promoter, into a suitable host cell line by an appropriate transfection method resulting in cells having the introduced sequences stably integrated into the genome. The calcium-phosphate co-precipitation method is an example of a transfection method which may be used according to the present invention.

The production of aPC may also include any method known in the art for the cultivation of said transformed cells, e.g. in a continuous or batchwise manner, and the expression of aPC e.g. constitutive or upon induction. In one specific example of the present invention the nucleic acid coding for aPC contained in the host organism is expressed via an expression mode selected from the group consisting of induced, transient, and permanent expression. Any expression system known in the art or commercially available can be employed for the expression of a recombinant nucleic acid encoding aPC, including the use of regulatory systems such as suitable, e.g. controllable, promoters, enhancers etc.

The production of aPC may also include any method known in the art for the isolation of aPC, e.g. from the culture medium or by harvesting the transformed cells. For example, aPC-producing cells can be identified by isolating single-cell derived populations i.e. cell clones, via dilution after transfection and optionally via addition of a selective drug to the medium. After isolation the identified cell clones may be cultivated until confluency in order to enable the measurement of aPC content of the cell culture supernatant by enzyme-linked immune-sorbent assay (ELISA) technique.

Additionally, the production of aPC may include any method known in the art for the purification of aPC, e.g. via anion exchange chromatography or affinity chromatography. In one preferred embodiment aPC can be purified from cell culture supernatants by semi-affinity calcium-dependent anion exchange chromatography, e.g. in an endotoxin-free system. The purified aPC may be analyzed by methods known in the art for analyzing recombinant aPC, e.g. the ELISA technique. In addition, aPC integrity and activity may be assessed. It is within the knowledge of a person skilled in the art to choose the optimal parameters, such as buffer system, temperature and pH for the respective detection system to be used.

In one specific example of the present invention, aPC transfected into a cell is expressed in a host cell type with the ability to perform posttranslational modifications. The ability to perform posttranslational modifications of aPC expressing host cell lines may be for example analyzed by mass-spectrometric analysis.

The host cell type used for the recombinant production of aPC may be any mammalian cell, preferably with the ability to perform posttranslational modifications of aPC. There is no particular limitation to the media, reagents and conditions used for culturing the cells in the cell culture used for the recombinant aPC production of including culturing the cells in a continuous or batchwise manner. The desired aPC which has been expressed by the cells of the and which, dependent on the transfection/vector-system used, is contained in the cells or secreted into the medium for culturing cells, can be isolated/recovered from the cell culture using methods known in the art, as mentioned herein before.

Within the scope of the invention, the term "cells" means a generic term and encompass the cultivation of individual cells, tissues, organs, insect cells, avian cells, mammalian cells, primary cells, continuous cell lines, stem cells and/or genetically engineered cells, such as recombinant cells expressing a heterologous aPC.

According to the present invention, the treatment of the transplant may include an incubation of the transplant with a composition comprising an effective amount of aPC. The composition comprising an effective amount of aPC may further comprise a physiologically acceptable carrier, diluent, salt, buffer, and/or excipient.

In a preferred embodiment of the present invention, the aPC is purified after activation in a suitable buffer, preferably in a buffer containing only Tris-Hcl (pH-7.5) and/or NaCl, most preferably in Tris-HCl (pH-7.5), 10mM NaCl. The composition comprising the aPC after purification may further comprise traces of thrombin from the source from which the aPC has been isolated. For example, in case the aPC has been isolated from human beings the composition comprising the purified aPC may further comprise traces of human thrombin. In a preferred embodiment of the present invention, the purified aPC is diluted for further use in PBS without calcium or magnesium.

In another preferred embodiment of the present invention, the incubation of the transplant with a composition comprising an effective amount of aPC is carried out in serum free cell culture medium which optionally may contain further additives. Any serum free cell culture medium which is commercially available is suitable for this purpose. In a preferred embodiment of the present invention serum free cell culture medium is AIM V medium (Life Technology). The serum free cell culture medium may contain any additive which does not prevent the treatment of the transplant with activated aPC. In a preferred embodiment, the additive is selected from the group consisting of L-glutamine, streptomycin sulfate, gentamicin sulfate, and human serum albumin. In a more preferred embodiment, the serum free cell culture medium contains L-glutamine, streptomycin sulfate at 50 µg/ml, and gentamicin sulfate at 10 µg/ml and human serum albumin. More preferably the additives added to the serum free cell culture medium consist of L-glutamine, streptomycin sulfate at 50 µg/ml, and gentamicin sulfate at 10 µg/ml and human serum albumin without any further additive added.

The term "preventing" as used herein relates to the prevention and/or eradication or amelioration of disease related symptoms and/or disease related disorders, of GVHD in a patient. The reduction of the above symptoms and disorders as well as the success of treatment or prevention of GVHD in a patient by use of aPC can be monitored using methods known in the art.

The prevention of GVHD can also be combined with any therapy known in the art for the prevention of GVHD. Accordingly, the present invention also relates to aPC for use in prevention of GVHD in a medicament which may also contain further active agents.

According to the present invention the terms "recipient" and "donor" as used herein does not underlie any specific limitation and includes all vertebrates.

In a preferred embodiment of the present invention, the present invention relates to aPC for use in preventing GVHD associated with bone marrow transplantation, wherein the recipient and the donor is a mammal. In a particularly preferred embodiment, the recipient and the donor is selected from the group consisting of human, mice, rats, dogs, cats, chimpanzees, and pigs, most preferably the recipient is human.

The donor and the recipient may be from the same species or from different species. Accordingly, the transplantation as defined herein may be an allogeneic transplantation or a xenogeneic transplantation. This includes a transplantation, wherein the donor and the recipient belong to the same species and the donor and/or the recipient are genetically altered to express at least one antigen derived from a different species and/or to express at least one component of an immune response derived from a different species. This also includes a transplantation, wherein the donor and the recipient belong to different species and the donor is genetically altered to express at least one antigen derived from the recipient's species and/or to express at least one component of an immune response derived from the recipient's species. This also includes a transplantation, wherein the donor and the recipient belong to different species and the recipient is genetically altered to express at least one antigen derived from the donor's species and/or to express at least one component of an immune response derived from the donor's species.

In a preferred embodiment of the present invention, the immune system of the recipient is impaired. Impairment of the immune system includes any reduction in speed, extent, diversity, and/or intensity of the immune response to an antigen. The impairment of the immune system may be caused by intrinsic factors within the body of the recipient or may be the caused by extrinsic factors to suppress the immune response of the recipient, like for example an immunosuppressive therapy. The impairment of the immune system may have been present before, during and/or after the transplantation.

In a particularly preferred embodiment of the present invention, the impairment of the immune system of the recipient is caused by an immunosuppressive treatment regiment, preferably radiation therapy and/or chemo-therapy, to remove the host's immune system prior to receiving the implant. In a more preferred embodiment of the present invention, the recipient's immune system is essentially inactive prior to receiving the implant.

In another preferred embodiment of the present invention, the recipient is immunologically disparate and/or histo-incompatible with regard to the donor. In a more preferred embodiment of the present invention, recipient and donor are less well matched for immunological markers than the ideal recipient and donor match, like for example with regard to major or minor histocompatibility antigens.

In a preferred embodiment of the present invention, the present invention relates to aPC for use in preventing GVHD associated with bone marrow transplantation, wherein the treatment of the transplant is carried out for 5 to 180 minutes at 33 to 38 °C, more preferably for 55 to 65 minutes at 36.5 to 37.5 °C, and most preferably for 60 minutes at 37 °C.

The term "transplant" as used herein includes any bone marrow transplant without any limitation. In a preferred embodiment of the present invention, the transplant is immune-competent. In a more preferred embodiment of the present invention, the transplant contains prior to the treatment with aPC viable and functional immune cells derived from the donor, particularly preferred functionally active T cells derived from the donor.

In view of the above, the present invention provides effective means for an effective prevention of GVHD following bone marrow transplantation.

The figures show:

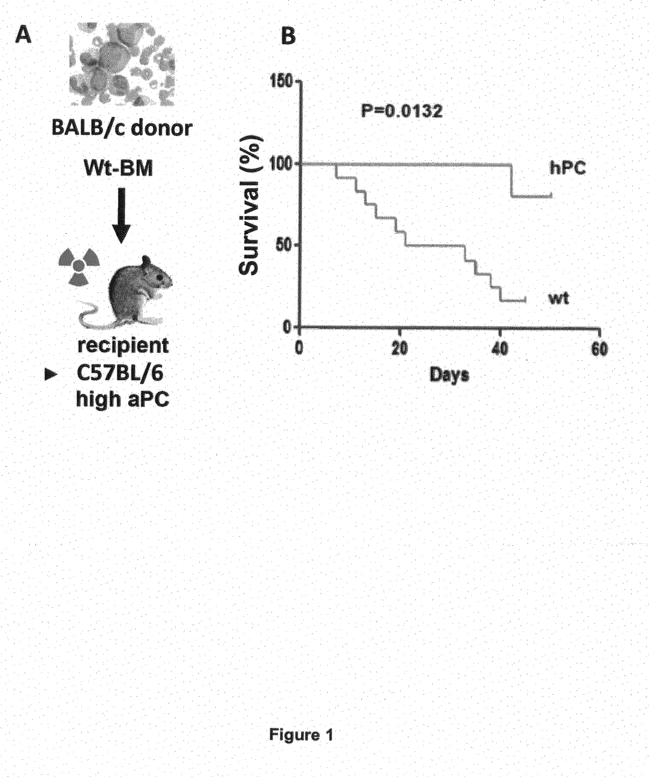

Figure 1: Survival of mice having GVHD: Activated human protein C protects mice against GVHD. Bone marrow transplantation was conducted in wild-type mice (wt) or mice expressing high levels of a human hyperactivatable PC variant (hPC mice), which results in high plasma levels of human activated protein C. The survival of hPC mice was significantly better than that of wt mice.

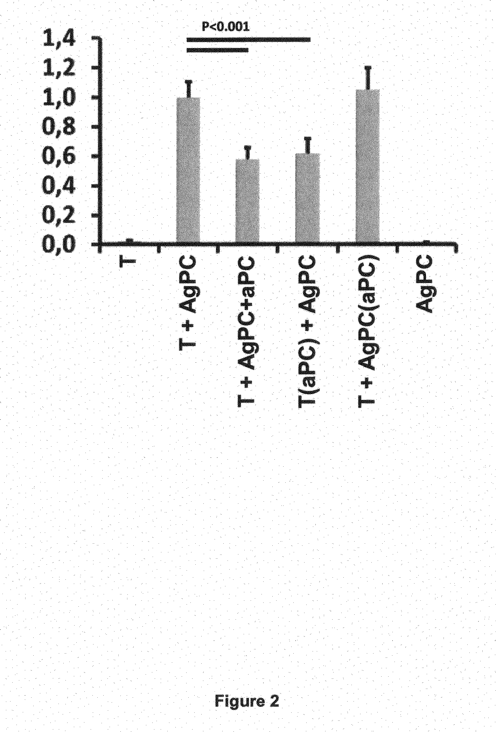

Figure 2: Incubation of T cells (T) with antigen presenting cells (AgPC) in the presence of activated Protein C (aPC, group: T + AgPC + aPC) reduces the T cell activation when measuring the proliferation. The same effect is obtained, when T cells are pre-incubated with aPC (group: T(aPC) + AgPC). However, pre-incubation of AgPC with aPC does not have any effect (T + AgPC (aPC)). As expected, the T cells which were not stimulated (T) or the radiated antigen presenting cells (AgPC) did not show enhanced proliferation.

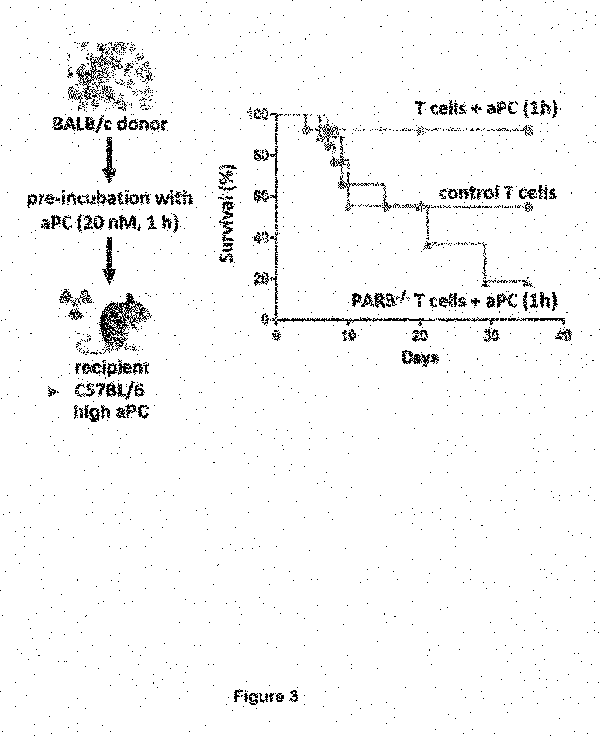

Figure 3: Pre-incubation of cells *ex vivo* and before transplantation is sufficient to avoid GVHD in mice after bone marrow transplantation.

The present invention will now be further illustrated in the following examples without being limited thereto.

### Example

### Example 1:

*In-vivo* transplantation (Fig. 1) included two groups of mice (wild-type control and hPC mice). hPC mice are a transgenic mouse line which constitutively expresses high levels of human hyperactivatable PC, resulting in high plasma levels of human aPC in these mice. In both cases the recipient mice were irradiated with 9 Gy 4-5hr before transplantation. Both groups were transplanted with 2 million allogenic T cells and in parallel with 5 million allogenic bone marrow cells *via* tail vein injection (the latter to restore the bone-marrow).

For *in-vitro* experiments (Fig, 2) co-culture of human T-cells with human antigen-presenting cells (AgPC) was done for 96h at 37°C, 5%CO₂. T-cells and AgPC were obtained from two genetically distinct donors (e.g. not twins). For "T+AgPC+aPC" conditions aPC (20nM) was added every 12h during the co-culture. For "T(aPC-20nM)" conditions pre-treatment of T cells was done with 20nM aPC for 1 hr at 37°C, 5%CO₂ and then pre-treated cells were washed sterile PBS and co-cultured with AgPC for 96h at 37°C, 5%CO₂. For "T(aPC-20nM)" conditions no aPC was added during the co-culture period. Proliferation was measured after thymidine incorporation for 16h in both the above conditions of co-culture.

*In-vivo* transplantation (Fig. 3) included two groups of mice. In both cases the recipient mice were irradiated with 11Gy 4-5hr before transplantation. The first group was transplanted with 1 million allogenic T cells without aPC pre-treatment, while the second group was transplanted with 1 million allogenic T cells pre-treated with aPC (20 nM) for 1 hr at 37°C; 5% CO2 in a humidified incubator. Pre-treated T cells were washed with PBS and then re-suspended in serum-free RPMI medium (without any antibiotics) and transplanted *via* tail vein injection. Both groups of mice were in parallel transplanted with 5 million allogenic bone marrow cells without any treatment (to restore the bone-marrow).

## Claims

1. An *ex vivo* method for the treatment of a bone marrow transplant, wherein the bone marrow transplant is treated *ex vivo* with activated Protein C (aPC) prior to implanting into the recipient and wherein the bone marrow transplant treatment is intended for use in preventing Graft versus Host Disease (GVHD) associated with transplantation.

2. The *ex vivo* method according to claim 1, wherein the recipient is a mammal.

3. The *ex vivo* method according to claim 2, wherein the recipient is selected from the group consisting of human, mice, rats, dogs, cats, chimpanzees, and pigs.

4. The *ex vivo* method according to any of the above claims, wherein the recipient is human.

5. The *ex vivo* method according to any of the above claims, wherein the treatment of the bone marrow transplant is carried out for 5 to 180 minutes at 33°C to 38°C.

6. The *ex vivo* method according to any of the above claims, wherein the bone marrow transplant is an allogeneic bone marrow transplant.

7. The *ex vivo* method according to any of the above claims, wherein the bone marrow transplant is a xenogeneic bone marrow transplant.

8. The *ex vivo* method according to any of the above claims, wherein the bone marrow transplant contains prior to the treatment with aPC functionally active T cell derived from the donor.

## Patentansprüche

1. *Ex-vivo*-Verfahren für die Behandlung eines Knochenmarktransplantats, wobei das Knochenmarktransplantat *ex vivo* vor dem-Implantieren in den Empfänger mit aktiviertem Protein C (aPC) behandelt wird und wobei die Behandlung des Knochenmarktransplantats zur Verwendung bei der Prävention von Graft-versus-Host-Krankheit (Graft-versus-host disease; GVHD) im Zusammenhang mit der Transplantation vorgesehen ist.

2. *Ex-vivo*-Verfahren nach Anspruch 1, wobei der Empfänger ein Säugetier ist.

3. *Ex-vivo*-Verfahren nach Anspruch 2, wobei der Empfänger aus der Gruppe, bestehend aus Mensch, Mäusen, Ratten, Hunden, Katzen, Schimpansen und Schweinen, ausgewählt ist.

4. *Ex-vivo*-Verfahren nach einem der vorstehenden Ansprüche, wobei der Empfänger ein Mensch ist.

5. *Ex-vivo*-Verfahren nach einem der vorstehenden Ansprüche, wobei die Behandlung des Knochenmarktransplantats für 5 bis 180 Minuten bei 33°C bis 38°C durchgeführt wird.

6. *Ex-vivo*-Verfahren nach einem der vorstehenden Ansprüche, wobei das Knochenmarktransplantat ein allogenes Knochenmarktransplantat ist.

7. *Ex-vivo*-Verfahren nach einem der vorstehenden Ansprüche, wobei das Knochenmarktransplantat ein xenogenes Knochenmarktransplantat ist.

8. *Ex-vivo*-Verfahren nach einem der vorstehenden Ansprüche, wobei das Knochenmarktransplantat vor der Behandlung mit aPC funktionell aktive T-Zellen, die von dem Spender stammen, enthält.

## Revendications

1. Procédé *ex vivo* pour le traitement d'un greffon de moelle osseuse, dans lequel le greffon de moelle osseuse est traité *ex vivo* avec de la protéine C activée (PCa) avant l'implantation chez le receveur et dans lequel le traitement du greffon de moelle osseuse est prévu pour une utilisation dans la prévention de la maladie du greffon contre l'hôte (MGCH) associée à une greffe.

2. Procédé *ex vivo* selon la revendication 1, dans lequel le receveur est un mammifère.

3. Procédé *ex vivo* selon la revendication 2, dans lequel le receveur est choisi dans le groupe constitué d'êtres humains, de souris, de rats, de chiens, de chats, de chimpanzés, et de porcs.

4. Procédé *ex vivo* selon l'une quelconque des revendications ci-dessus, dans lequel le receveur est un être humain.

5. Procédé *ex vivo* selon l'une quelconque des revendications ci-dessus, dans lequel le traitement du greffon de moelle osseuse est réalisé pendant 5 à 180 minutes à 33 °C à 38 °C.

6. Procédé *ex vivo* selon l'une quelconque des revendications ci-dessus, dans lequel le greffon de moelle osseuse est un greffon de moelle osseuse allogénique.

7. Procédé *ex vivo* selon l'une quelconque des revendications ci-dessus, dans lequel le greffon de moelle osseuse est un greffon de moelle osseuse xénogénique.

8. Procédé *ex vivo* selon l'une quelconque des revendications ci-dessus, dans lequel le greffon de moelle osseuse contient avant le traitement avec de la PCa des lymphocytes T fonctionnellement actifs dérivés du donneur.
